# EUROPÄISCHE PATENTSCHRIFT

(11) **EP 2 546 839 B1**
(45) Veröffentlichungstag und Bekanntmachung des Hinweises auf die Patenterteilung: **04.11.2015**
(21) Anmeldenummer: 12163203.8
(22) Anmeldetag: 04.04.2012
(51) Int. Cl.: G21G 1/00, B01D 15/16, B01D 15/18, B01D 15/36

(54) **Verfahren zur Herstellung trägerfreier hochreiner 177Lu-Verbindungen sowie trägerfreie 177Lu-Verbindungen**
Method for producing carrier-free extremely pure 177Lu compounds and carrier-free 177Lu compounds
Procédé de fabrication de liaisons 177Lu à pureté élevée sans porteur ainsi que les liaisons 177Lu sans porteur

(30) Priorität: 15.07.2011 DE 102011051868
(43) Veröffentlichungstag der Anmeldung: 16.01.2013
(73) Patentinhaber: ITM Isotopen Technologien München AG, 85748 Garching (DE)
(72) Erfinder: Marx, Sebastian, 85356 Freising (DE); Harfensteller, Mark, 85716 Unterschleißheim (DE); Zhernosekov, Konstantin, 80805 München (DE); Nikula, Tuomo, 85521 Ottobrunn (DE)
(74) Vertreter: Winter, Brandl, Fürniss, Hübner, Röss, Kaiser, Polte - Partnerschaft mbB

(56) Entgegenhaltungen:
- JP-A- 2010 223 827
- US-B1- 6 716 353
- HASHIMOTO, MATSUOKA, H UCHIDA: "Production of no-carrier-added 177Lu via the 176Yb(n, gamma)177Yb -> 177Lu process", JOURNAL OF RADIOANALYTICAL AND NUCLEAR CHEMISTRY, Bd. 255, Nr. 3, 1. März 2003 (2003-03-01), Seiten 575-579, XP002678871, ISSN: 0236-5731

## Beschreibung

Die vorliegende Erfindung betrifft ein Verfahren zur Herstellung im Wesentlichen trägerfreier hochreiner ¹⁷⁷Lu-Verbindungen für medizinische Zwecke, und/oder diagnostische Zwecke, gemäß Anspruch 1 und Anspruch 10 sowie eine Verwendung einer Kationenaustauschchromatographie-Säule zum Herstellen von hochreinen, trägerfreien ¹⁷⁷Lu-Verbindungen gemäß Anspruch 12.

Durch erfolgversprechende klinische Ansätze in der Radionuklidtherapie sowie - diagnostik wächst die Nachfrage nach dem Reaktornuklid ¹⁷⁷Lu weltweit, Als niederenergetischer β-Strahler mit einer relativ kurzen Halbwertszeit von T_{½} = 6,71 Tagen bildet ¹⁷⁷Lu ein ausgezeichnetes Vehikel zur gezielten Deponierung großer Energiemengen in kleinen Volumina. Diese physikalischen Eigenschaften werden in Form der Radioimmun- und Peptidrezeptor-Radionuklidtherapie mit vielversprechenden Ergebnissen vorwiegend in der Onkologie insbesondere zur Behandlung und Diagnose von Tumoren eingesetzt.

¹⁷⁷Lu kann bekanntlich durch folgende Kernreaktionen erzeugt werden:

Direktes Verfahren: ¹⁷⁶Lu(n,γ)¹⁷⁷Lu (1)

Indirektes Verfahren: ¹⁷⁶Yb(n,γ)¹⁷⁷Yb →¹⁷⁷Lu (2)

Die Kernreaktion (1) stellt eine Neutroneneinfangreaktion an ¹⁷⁶Lu dar, welche in letzter Konsequenz zu geträgertem ¹⁷⁷Lu (¹⁷⁷Lu carrier added [¹⁷⁷Lu c.a.]) und dadurch zu begrenzter Produktqualität in Form von deutlich geringerer spezifischer Aktivität führt. Dadurch ist bei der Markierung von Biomolekülen mit ¹⁷⁷Lu die pro Menge an Biomolekül gebundene Aktivität deutlich geringer. Dies führt bei begrenzter Rezeptorzahl auf der Tumoroberfläche zu schlechteren Therapieergebnissen oder Nebenwirkungen. Durch die Bestrahlung von ¹⁷⁶Lu wird zusätzlich das in medizinischer und strahlenschutztechnischer Hinsicht unerwünschte, langlebige metastabile Radionuklid ^{177m}Lu (T½ = 160,1 d) erzeugt. Der Anteil des ^{177m}Lu kann je nach Bestrahlungsparameter bis zu 0,1 % der ¹⁷⁷Lu-Aktivität betragen. Im Hinblick auf die Anwendung am Menschen und angesichts der hohen zu produzierenden Gesamtaktivitäten ist diese Verunreinigung kritisch zu sehen. Im Rahmen der Behandlungen besteht ein anhaltendes erhöhtes Risiko der ^{177m}Lu-Freisetzung in die Umwelt, bedingt durch die lange Halbwertszeit des Nuklids und die renale Ausscheidung von mit den Lu-Isotopen behandelten Patienten. Somit steht der Verbraucher in der Klinik vor dem Problem der sicheren Handhabung und Entsorgung der Restmengen eines langlebigen Nuklids, welches durch die in Kliniken übliche Lagerung der radioaktiven Abfälle kaum zu lösen ist.

Wie eingangs erwähnt, weist derzeit auf dem Markt erhältliches, geträgertes ¹⁷⁷Lu diverse Nachteile gegenüber dem trägerfreien ¹⁷⁷Lu auf. Durch die bisher bessere Verfügbarkeit des ¹⁷⁷Lu c.a. wird dieses trotz seiner Nachteile von vielen Kliniken dennoch bevorzugt.

Das derzeit auf dem Markt erhältliche ¹⁷⁷Lu wird im Wesentlichen von drei Anbietern vertrieben. Sämtliche Anbieter produzieren ¹⁷⁷Lu über dieselbe Route, nämlich über obige Kernreaktion (1) direkt aus ¹⁷⁶Lu.

Dies führt zu den erwähnten Problemen.

Die attraktivere, medizinisch und kommerziell sinnvollere aber technisch anspruchsvollere Option ist somit die Herstellung von trägerfreiem ¹⁷⁷Lu über die indirekte Kernreaktion (2). Eine derartige Kernreaktion kann beispielsweise an Hochfluss-Neutronenquellen genutzt werden, um trägerfreies ¹⁷⁷Lu herzustellen. Durch die Bestrahlung von ¹⁷⁶Yb wird das kurzlebige Radioisotop ¹⁷⁷Yb (T½ = 1.9 h) erzeugt, welches zu ¹⁷⁷Lu zerfällt.

In diesem Fall ist das erwünschte Nuklid ¹⁷⁷Lu ein Nuklid eines anderen Elements als das Element des Targetnuklids ¹⁷⁶Yb und kann daher chemisch in trägerfreier Form (¹⁷⁷Lu no carrier added [¹⁷⁷Lu n.c.a.]) isoliert werden, sofern eine quantitative Abtrennung von Yb-Nukliden möglich ist. Da durch den Zerfall des Nuklids ¹⁷⁷Yb kein ^{177m}Lu entsteht, kann das ¹⁷⁷Lu mit einer sehr hohen radioisomerischen und radionuklidischen Reinheit hergestellt werden.

Der Nachteil bei der Wahl dieser Strategie ist jedoch das notwendige radiochemische Verfahren zur Trennung des Yb(makro)/¹⁷⁷Lu(mikro)-Systems. Da das Ziel- und das Targetnuklid zwei benachbarte Elemente in der Lanthanidenreihe sind, bleibt die Abtrennung auf Grund ihrer chemischen Ähnlichkeit eine große Herausforderung.

Ein Lösungsansatz für die oben angesprochene Trennproblematik findet sich in dem Patent US 6,716,353 B1, welches die Abtrennung von ¹⁷⁷Lu n.c.a. von Ytterbium bei Beschreitung des indirekten Weges gemäß obiger Gleichung (2) beschreibt, um so ein ¹⁷⁷Lu mit hoher spezifischer Aktivität zu erzeugen. Dabei wird zuerst Ytterbium durch den Einsatz mäßig konzentrierter Mineralsäuren von einem LN-Harz, welches Di-(2-ethylhexyl)-orthophosphorsäure (HDEHP) als Extraktanten umfasst (Ln Resin der Firma Eichrom). Gemäß dem Verfahren der US 6,716,353 B1 wird mit mäßig konzentrierter Salzsäure zuerst das Ytterbium von einer LN-Harz enthaltenden chromatographischen Säule eluiert und anschließend das ¹⁷⁷Lu durch den Einsatz höher konzentrierter Salzsäure erhalten.

Aufgrund der Tatsache, dass mikroskopische Mengen ¹⁷⁷Lu von makroskopischen Mengen Ytterbium zu trennen sind, ist der Nachteil dieses Verfahrens des Standes der Technik darin zu sehen, dass gemäß US 6,716,353 B1 zuerst die makroskopische in extremen Überschuss vorliegende Komponente eluiert wird. Da ein Verteilen des Ytterbiums durch Tailing am Ende des Peaks eine Folge des extraktionschromatographischen Systems ist, muss der Prozess mehrfach wiederholt werden, um ¹⁷⁷Lu n.c.a. in der entsprechenden Qualität zu erhalten, wobei unweigerlich ein nicht zu vernachlässigender Restgehalt an ¹⁷⁶Yb systembedingt im Lu-Eluat verbleibt. Zudem werden gemäß dem Stand der Technik der US 6,716,353 B1 lediglich Aktivitätsmengen im MBq-Bereich erhalten. Da es sich bei dem gemäß US 6,716,353 B1 offenbarten Verfahrens um ein extraktionschromatographisches Verfahren handelt, bedeutet dies zudem, dass ein Extraktant an die Oberfläche des Säulenmaterials adsorbiert ist, der naturgemäß zum Teil mit dem erwünschten ¹⁷⁷Lu eluiert wird und das Produkt somit zusätzlich chemisch verunreinigt. Zudem ist für die Elution des ¹⁷⁷Lu eine große Menge konzentrierter Salzsäure erforderlich, in der das Produkt anschließend vorliegt. Darüber hinaus ist das in der US 6,716,353 B1 beschriebene Verfahren sehr langwierig und benötigt eine Prozesszeit von über 16 Stunden auf einer einzigen Säule. Bei den erforderlichen Wiederholungsschritten dauert die Produktion somit mehrere Tage.

Sehr hohe medizinische Anforderungen an die Qualität des Nuklids ¹⁷⁷Lu erschweren somit den Herstellungsprozess und dadurch dessen Machbarkeit.

Eine erfolgreiche Anwendung des Radionuklids ¹⁷⁷Lu ist allerdings durch die produktionstechnisch erzielbare spezifische Aktivität des Nuklids [Bq/mg] und dessen Reinheit bestimmt. Eine hohe spezifische Aktivität des Radionuklids ist notwendig, um eine möglichst hohe spezifische Aktivität und damit optimale, eingesetzte Mengen eines entsprechenden Radiopharmakons zu erreichen. Wird keine hohe spezifische Aktivität und Reinheit erreicht, so kann dies unter anderem zu einer negativen Beeinflussung der Herstellung des Radiopharmakons oder der Qualität des Radiopharmakons selbst führen.

Darüber hinaus offenbart die JP 2010 223827 ein Trenn- bzw. Reinigungsverfahren für ¹⁷⁷Lu aus ¹⁷⁶Yb₂O₃ unter Verwendung einer HPLC, wobei eine salzsaure Lösung des bestrahlten ¹⁷⁶Yb₂O₃ zuvor über einen Kationen- und Chelataustausch gereinigt wird und sich des weiteren nach dem Kationaustauschschritt ein Aninonenaustauschschritt zum Entfernen von Fe anschließt.

Ferner beschreiben Hashimoto et al.: "Productin of no-carrier-added 177Lu via the 176Yb(n,γ)177Yb → 177Lu process", J Radioanal Nucl Ch, Bd. 255, Nr. 3, 1. März 2003, 575-579 ein Verfahren zur Herstellung im Wesentlichen trägerfreier hochreiner ¹⁷⁷Lu-Verbindungen für medizinische Zwecke aus mit thermischen Neutronen bestrahlten ¹⁷⁶Yb-Verbindungen, wobei die Endprodukte der Neutronenbestrahlung, die im Wesentlichen eine Mischung aus ¹⁷⁷Lu und ¹⁷⁶Yb im Massenverhältnis von ca. 1:10² bis 1:10¹⁰ enthalten, als Ausgangsmaterialien eingesetzt werden, wobei in Wasser unlösliches Yb₂O₃ mittels HCl in eine lösliche Form überführt werden.

Ausgehend vom nächstkommenden Stand der Technik der Hashimoto et al. ist es daher die objektive technische Aufgabe der vorliegenden Erfindung, ein Verfahren zur Verfügung zu stellen, um hochreines trägerfreies ¹⁷⁷Lu (non carrier added [n.c.a.] ¹⁷⁷Lu) in industriellem Maßstab für medizinische Zwecke zur Verfügung zu stellen.

Die Lösung dieser Aufgabe erfolgt in verfahrenstechnischer Hinsicht durch die Merkmale der Ansprüche 1 und 10 und im Hinblick auf eine Verwendung durch die Merkmale des Anspruchs 12.

Insbesondere betrifft die vorliegende Erfindung ein Verfahren zur Herstellung im Wesentlichen trägerfreier hochreiner ¹⁷⁷Lu-Verbindungen für therapeutische und/oder diagnostische Zwecke aus mit thermischen Neutronen bestrahlten ¹⁷⁶Yb-Verbindungen, wobei die Endprodukte der Neutronenbestrahlung, die im Wesentlichen eine Mischung aus den Nukliden ¹⁷⁷Lu und ¹⁷⁶Yb im Massenverhältnis von ca. 1:10² bis 1:10¹⁰ enthalten, als Ausgangsmaterialien eingesetzt werden, wobei in Wasser unlösliche Ausgangsmaterialien gegebenenfalls mittels Mineralsäuren und/oder erhöhter Temperatur in eine lösliche Form überführt werden, und wobei das Verfahren folgende Schritte umfasst:
a) Beladen einer ersten Säule, die mit einem Kationenaustauschermaterial gepackt ist, mit den in Mineralsäure gelösten Ausgangsmaterialien, die ¹⁷⁷Lu und ¹⁷⁶Yb im Massenverhältnis von ca. 1:10² bis 1:10¹⁰ enthalten; Austauschen der Protonen des Kationenaustauschermateriales gegen Ammoniumionen unter Einsatz einer NH₄Cl-Lösung; und Spülen des Kationenaustauschermateriales der ersten Säule mit Wasser;
b) Verbinden des Ausgangs der ersten Säule mit dem Eingang einer zweiten Säule, die ebenfalls mit einem Kationenaustauschermaterial gepackt ist;
c) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner an den Eingang der ersten Säule, um ¹⁷⁷Lu-Verbindungen von der ersten und der zweiten Säule zu eluieren;
d) Erfassen der Radioaktivitätsdosis am Ausgang der zweiten Säule, um eine Elution von ¹⁷⁷Lu-Verbindungen zu erkennen; und Auffangen eines ersten ¹⁷⁷Lu-Eluates vom Ausgang der zweiten Säule in einem Gefäß; und Protonieren des Komplexbildners, um diesen für die Komplexbildung mit ¹⁷⁷Lu-Ionen unwirksam zu machen;
e) Beladung einer finalen Trennsäule, die mit einem Kationenaustauschermaterial gepackt ist, durch kontinuierliche Weiterleitung des sauren ¹⁷⁷Lu-Eluates aus Schritt d) zum Eingang der finalen Trennsäule; Auswaschen des Komplexbildners mit verdünnter Mineralsäure einer Konzentration von kleiner als ca. 0,1 M; Entfernen von Fremdmetallionenspuren aus der ¹⁷⁷Lu-Lösung durch Waschen des Kationenaustauschermateriales der finalen Trennsäule mit Mineralsäure unterschiedlicher Konzentrationen im Bereich von ca. 0, 1 bis 2,5M;
f) Eluieren der ¹⁷⁷Lu-Ionen von der finalen Trennsäule mittels einer hochkonzentrierten Mineralsäure von ca. 3M bis 12M; Auffangen des hochreinen ¹⁷⁷Lu-Eluates in einer Verdampfereinrichtung und Entfernen der Mineralsäure durch Verdampfen.

Die beschriebene Ausführungsform kann durch Wiederholung des Trennverfahrens mit dem Komplexbildner α-Hydroxyisobutyrat und den beschriebenen Säulensystemen beliebig oft wiederholt werden, wie in folgender Ausführungsform beispielhaft beschrieben wird:
Eine alternative Ausführungsform des erfindungsgemäßen Verfahrens ist ein Verfahren zur Herstellung im Wesentlichen trägerfreier hochreiner ¹⁷⁷Lu-Verbindungen für medizinische Zwecke aus mit thermischen Neutronen bestrahlten ¹⁷⁶Yb-Verbindungen, wobei die Endprodukte der Neutronenbestrahlung, die im Wesentlichen eine Mischung aus den Nukliden ¹⁷⁷Lu und ¹⁷⁶Yb im Massenverhältnis von ca. 1:10² bis 1:10¹⁰ enthalten, als Ausgangsmaterialien eingesetzt werden, wobei in Wasser unlösliche Ausgangsmaterialien mittels Mineralsäuren und/oder erhöhter Temperatur in eine lösliche Form überführt werden, und wobei das Verfahren folgende Schritte umfasst:
   a) Beladen einer ersten Säule, die mit einem Kationenaustauschermaterial gepackt ist, mit den in Mineralsäure gelösten Ausgangsmaterialien, die ¹⁷⁷Lu und ¹⁷⁶Yb im Massenverhältnis von ca. 1:10² bis 1:10¹⁰ enthalten; Austauschen der Protonen des Kationenaustauschermateriales gegen Ammoniumionen unter Einsatz einer NH₄Cl-Lösung; und Spülen des Kationenaustauschermateriales der ersten Säule mit Wasser;
   b) Verbinden des Ausgangs der ersten Säule mit dem Eingang einer zweiten Säule, die ebenfalls mit einem Kationenaustauschermaterial gepackt ist;
   c) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner, an den Eingang der ersten Säule;
   d) Erfassen der Radioaktivitätsdosis am Ausgang der zweiten Säule, um eine Elution von ¹⁷⁷Lu-Verbindungen zu erkennen; und Auffangen eines ersten ¹⁷⁷Lu-Eluates vom Ausgang der zweiten Säule in einem Gefäß; und Protonieren des Komplexbildners, um diesen für die Komplexbildung mit ¹⁷⁷Lu-Ionen unwirksam zu machen;
   e) kontinuierliche Weiterleitung des sauren ¹⁷⁷Lu-Eluates aus Schritt d) zum Eingang einer dritten Säule, die mit einem Kationenaustauschermaterial gepackt ist, wobei das Kationenaustauschermaterial durch die Beladung mit dem sauren ¹⁷⁷Lu-Eluat in protonierter Form vorliegt; Austauschen der Protonen des Kationenaustauschermateriales gegen Ammoniumionen unter Einsatz einer NH₄Cl-Lösung; und Spülen des Kationenaustauschermateriales der dritten Säule mit Wasser;
   f) Verbinden des Ausgangs der dritten Säule mit dem Eingang einer vierten Säule, die mit einem Kationenaustauschermaterial gepackt ist;
   g) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner, an den Eingang der dritten Säule;
   h) Erfassen der Radioaktivitätsdosis am Ausgang der vierten Säule, um eine Elution der ¹⁷⁷Lu-Verbindungen zu erkennen; und Auffangen eines zweiten ¹⁷⁷Lu-Eluates vom Ausgang der vierten Säule in einem Gefäß; und Protonieren des Komplexbildners, um diesen für die Komplexbildung mit ¹⁷⁷Lu-Ionen unwirksam zu machen;
   i) Beladung einer finalen Säule, die mit einem Kationenaustauschermaterial gepackt ist, durch kontinuierliche Weiterleitung des sauren ¹⁷⁷Lu-Eluates aus Schritt h) zum Eingang der finalen Säule; Auswaschen des Komplexbildners mit verdünnter Mineralsäure; Entfernen von Fremdmetallionenspuren aus der ¹⁷⁷Lu-Lösung durch Waschen des Kationenaustauschermateriales der finalen Säule mit Mineralsäure unterschiedlicher Konzentrationen im Bereich von ca. 0,01 bis 2,5M;
   j) Eluieren der ¹⁷⁷Lu-Ionen von der finalen Trennsäule mittels einer konzentrierten Mineralsäure von ca. 1 M bis hin zu ca. 12 M; Auffangen des hochreinen ¹⁷⁷Lu-Eluates in einer Verdampfereinrichtung und Entfernen der Mineralsäure durch Verdampfen.

Zwar offenbart der Stand der Technik gemäß Hollemann-Wieberg, "Lehrbuch der Anorganischen Chemie", Walter de Gruyter Verlag, Berlin-New York, 102. Auflage, 2007, Seite 1932 bis 1933 schon seit langem das Grundprinzip der Trennung von Lanthanoiden und insbesondere von dreiwertigen Lanthanoiden auf der Basis von Kationenaustausch und Komplexierung, jedoch gilt dies nur für ein Vorliegen ähnlicher Mengen an Lanthanoiden und nicht für Massenverhältnisse, bei denen das erwünschte Lanthanoidkation aus einem millionenfachen massebezogenen Überschuss eines anderen Lanthanoids in höchster Reinheit isoliert werden muß. Außerdem ergibt sich selbst dann gemäß dem Stand der Technik von Hollemann-Wieberg, insbesondere aus Fig. 393 eine nur ungenügende Trennschärfe zwischen Lu und Yb, da beide Peaks signifikant überlappen, wenn die Elution der Lanthanoiden aus dem Ionenaustauscherharz Dowex-50 mit Ammonium-α-hydroxyisobutyrat in einer Mischung der Lanthanoiden Eu, Gd, Tb, Dy, Ho, Er, Tm, Yb und Lu durchgeführt wird.

Im Gegensatz zu den im Stand der Technik beschriebenen Verfahren ist es mit der vorliegenden Erfindung erstmals möglich, industriell relevante Mengen trägerfreien ¹⁷⁷Lu in hochreiner Form so herzustellen, dass eine unmittelbare Weiterverarbeitung wie beispielsweise Kopplung an Biomoleküle zur Herstellung von Radiopharmazeutika erfolgen kann. Dies liegt insbesondere daran, dass die Reinheits- und Sterilitätsanforderungen and das erhaltene ¹⁷⁷Lu-Produkt gegeben sind und das Verfahren vollständig mit den EU-GMP-Richtlinien kompatibel ist.

Ein besonderer Vorteil des erfindungsgemäßen Herstellungsverfahrens ist, dass Ytterbium in Gramm-Mengen verarbeitet werden kann. Dies ermöglicht die Produktion von mehreren Terabecquerel (TBq) ¹⁷⁷Lu n.c.a. pro Produktionslauf. Der Herstellungsprozess ermöglicht somit erstmals die Produktion von Milligrammmengen des Radionuklids ¹⁷⁷Lu n.c.a., das sich aufgrund seiner chemischen und radiochemischen Reinheit für den Einsatz in der Nuklearmedizin bzw. -diagnostik eignet.

Ein weiterer Vorteil des erfindungsgemäßen Verfahrens liegt darin begründet, dass es innerhalb von etwa 10 Stunden bis zum Erhalt des Endproduktes durchgeführt werden kann.

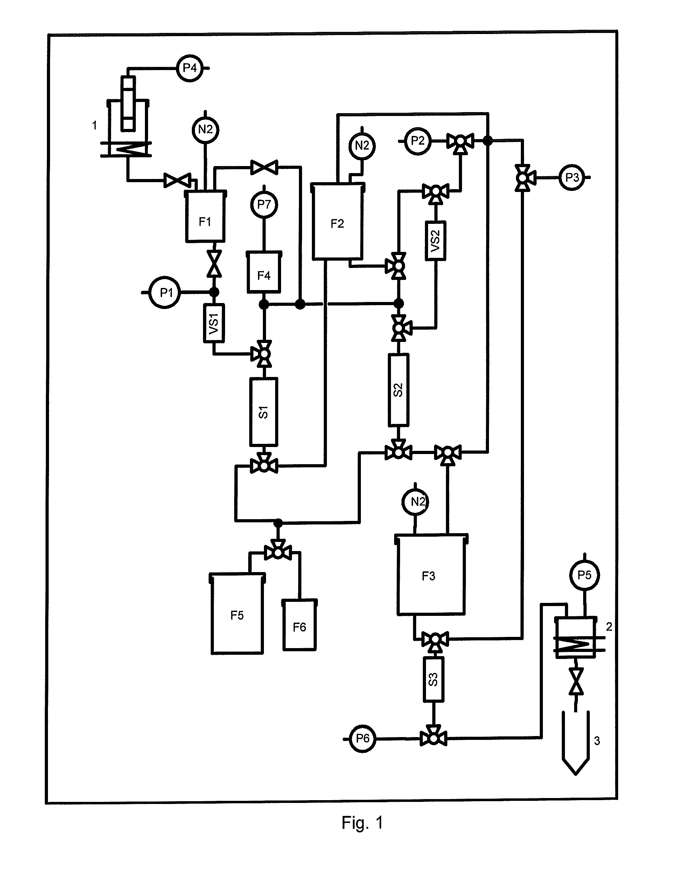

Dies kann beispielsweise auf mehrere Faktoren zurückgeführt werden. Zum einen laufen viele Prozesse parallel ab und so können durch die in einer bevorzugten Ausführungsform verwendeten Vorsäulensysteme VS1 und VS2 (vgl. Fig. 1) die Prozesse der jeweils nachfolgenden Trennungen schon während der laufenden vorhergehenden Trennung gestartet werden. Des Weiteren können die Gradienten der Pumpen auf hohe Trennfaktoren und kurze Retentionszeiten für ¹⁷⁷Lu optimiert werden. Werden z. B. Vorsäulen verwendet, ermöglicht dies beispielsweise die Beladung der Kationenaustauschermaterialien mit sauren bzw. angesäuerten Lösungen, die in dieser Form an und für sich nicht für eine Trennung optimal geeignet wären. Damit können aufwändige Prozessschritte wie Verdampfen oder Neutralisieren wenigstens weitgehend entfallen. Auch wird die Korrosion der Produktionsanlage dadurch vermieden, da somit keine aggressiven Dämpfe durch zusätzliche Verdampfungsschritte entstehen. Zudem wird das Kontaminationsrisiko deutlich reduziert. Durch das Spülen der Vorsäulen können Verunreinigungen aus dem System entfernt und bei Bedarf geeignet entsorgt oder einer Wiederverwendung zugeführt werden.

Durch die Verwendung von Vorsäulen wird im Allgemeinen die Trennung des erwünschten ¹⁷⁷Lu von Yb verbessert und durch einen finalen Aufreinigungsschritt mit einer weiteren Säule wird die Qualität weiter gesteigert, da somit auch noch Spuren von Fremdmetallen aus dem ¹⁷⁷Lu-Produkt entfernt werden können. Darüber hinaus ermöglicht das erfindungsgemäße Verfahren die Bereitstellung eines bereits sterilen Endproduktes, welches zudem praktisch toxinfrei ist und welches unmittelbar zur radiopharmazeutischen Weiterverarbeitung, z. B. Kopplung an Proteine, eingesetzt werden kann.

Die Dimensionierung solcher Vorsäulen und Trennsäulen hinsichtlich ihrer geometrischen Abmessungen sowie deren Dimensionsverhältnisse untereinander ist dem Fachmann wohl bekannt.

Es ist bevorzugt, das erfindungsgemäße Verfahren gemäß folgender alternativen Ausführungsform durchzuführen: Es werden zwischen den Schritten d) und f) gemäß Anspruch 1 die folgenden Schritte zusätzlich durchgeführt:
d.1) kontinuierliche Weiterleitung bei gleichzeitiger Ansäuerung des ¹⁷⁷Lu-Eluates aus Schritt d) zum Eingang einer dritten Säule, die mit einem Kationenaustauschermaterial gepackt ist, wobei das Kationenaustauschermaterial durch die Beladung mit dem sauren ¹⁷⁷Lu-Eluat in protonierter Form vorliegt; Austauschen der Protonen des Kationenaustauschermateriales gegen Ammoniumionen unter Einsatz einer NH₄Cl-Lösung; und Spülen des Kationenaustauschermateriales der dritten Säule mit Wasser;
d.2) Verbinden des Ausgangs der dritten Säule mit dem Eingang einer vierten Säule, die mit einem Kationenaustauschermaterial gepackt ist;
d.3) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner, an den Eingang der dritten Säule, um ¹⁷⁷Lu-Verbindungen von der dritten und der vierten Säule zu eluieren;
d.4) Erfassen der Radioaktivitätsdosis am Ausgang der vierten Säule, um eine Elution der ¹⁷⁷Lu-Verbindungen zu erkennen; und Auffangen eines zweiten ¹⁷⁷Lu-Eluates vom Ausgang der dritten Säule in einem Gefäß; und Protonieren des Komplexbildners, um diesen für die Komplexbildung mit ¹⁷⁷Lu-Ionen unwirksam zu machen.

Der Vorteil dieser Vorgehensweise liegt darin, daß mit jeweils zwei in Prozessrichtung hintereinander geschalteten Säulenpaaren jeweils eine Vorsäule und eine Trennsäule zur Verfügung steht. Nach Durchlaufen des zweiten Paares Vorsäule und Trennsäule wird das doppelt aufgereinigte ¹⁷⁷Lu-Eluat dann auf eine finale Trennsäule gegeben und noch von weiteren Metallspuren befreit. Darüber hinaus bietet das Vorsäulen/Trennsäulenkonzept noch den Vorteil, dass es einen Säulenauftrag von sauren bzw. angesäuerten Lösungen, die an und für sich nur bedingt für eine Trennung geeignet wären, ermöglicht. Die eigentliche scharfe Trennung erfolgt dann erst in der Trennsäule, also beispielsweise der zweiten und/oder vierten Säule. Ein weiterer Vorteil ist die verkürzte Prozesszeit aufgrund einer schnelleren möglichen Beladung der kleineren Vorsäulen.

Selbstverständlich ist dem Fachmann wohl bekannt, dass bei Bedarf auch mehr als zwei Vorsäulen/Trennsäulen-Paare eingesetzt werden können.

In Bezug auf ein Recycling der eingesetzten Yb-Materialien und einer reduzierten Prozesszeit ist es von Vorteil, wenn nach der Elution der ¹⁷⁷Lu-Verbindungen in den Schritten und d) und d.4) die erste und zweite Säule sowie die dritte und die vierte Säule mit höheren Komplexbildnerkonzentrationen gewaschen werden, um Yb-Ionen von dem Kationenaustauschermaterial zu eluieren; und erhaltene Yb-Eluate, welche im Wesentlichen ¹⁷⁶Yb-Ionen enthalten, zum Zwecke der Wiederverwendung als Ausgangsmaterial für die ¹⁷⁷Lu-Herstellung gesondert gesammelt werden.

Als geeignete Mineralsäuren zum Ansäuern des ¹⁷⁷Lu-Eluats haben sich die folgenden herausgestellt: HNO₃, HCl, H₂SO₄, HF, sowie organische Säuren wie bspw. Essigsäure.

Sofern ¹⁷⁷Lu-Verbindungen aus in Wasser unlöslichen ¹⁷⁶Yb-Oxiden gewonnen werden sollen, ist es möglich und bevorzugt, diese Oxide beispielsweise durch den Einsatz von 1 M bis 12M HNO₃ oder anderen oxidierenden Säuren in eine wasserlösliche Form zu überführen.

Typischerweise erfolgt die Beladung der Kationenaustauschermaterialien mit einer Säurekonzentration von 0,01 M bis 2M HNO₃, HCl oder anderen anorganischen und/oder organischen Säuren

Als besonders geeignet hat sich ein Kationenaustauschermaterial herausgestellt, welches ausgewählt wird aus der Gruppe bestehend aus: makroporösen und gelartigen Kationenaustauschharzen auf Polystyrolbasis oder auf Basis anderer organischer Polymere sowie Kationenaustauscherharzen auf Silikatbasis.

Anders als im Stand der Technik können vorzugsweise Grammmengen an Yb-Ausgangsmaterialien eingesetzt werden und bis zu Milligrammmengen an ¹⁷⁷Lu produziert werden.

Typischerweise liegen die Ausbeuten bei mehren TBq ¹⁷⁷Lu und es können spezifische Aktivitäten von ca. 3,9 TBq ¹⁷⁷Lu /mg Lutetium erhalten werden, welche dicht an die theoretische physikalische Grenze von 4 TBq ¹⁷⁷Lu /mg ¹⁷⁷Lu herankommen.

Aus Strahlenschutzgründen wie auch aus arzneimittelrechtlichen Gründen führt man das vorliegenden Verfahren in einer Heißen Zelle mit mindestens der Reinraumklasse C nach EU-GMP-Regeln durch.

Um die pharmazeutische Qualität des trägerfreien ¹⁷⁷Lu-Produktes zu gewährleisten und um die Herstellungserlaubnis zu erlangen, wurde die chromatographische Vorrichtung zur Durchführung des erfindungsgemäßen Verfahrens in eine Reinraumumgebung überführt. Darüber hinaus ermöglicht die Verwendung einer Heißen Zelle auch die Durchführung des erfindungsgemäßen Verfahrens in Form eines semi-automatischen bzw. vollautomatischen Prozesses.

Schlussendlich führt das erfindungsgemäße Verfahren zu einer trägerfreien ¹⁷⁷Lu-Verbindung (Lu-177 n.c.a), wobei die ¹⁷⁷Lu-Verbindung erhältlich ist nach wenigstens einem der Verfahren gemäß Anspruch 1 bis 11.

Ein besonderer Vorteil der trägerfreien ¹⁷⁷Lu-Verbindung ist es, dass sie unmittelbar - also ohne weitere Aufreinigung und/oder Sterilisation - für den radiopharmazeutischen Einsatz geeignet ist.

Mit der nach dem erfindungsgemäßen Verfahren hergestellten ¹⁷⁷Lu-Verbindung lässt sich ein Markierungsverhältnis von mehr als 400 MBq ¹⁷⁷Lu pro µg Peptid oder Polypeptid oder sonstigen Biomolekülen erreichen.

Ein weitere Vorteil der erfindungsgemäß hergestellten trägerfreien ¹⁷⁷Lu-Verbindung ist es, dass sie auch noch nach mehreren Wochen nach ihrer Herstellung für die Markierung von Peptiden, Polypeptiden, Antikörpern oder sonstigen Biomolekülen eingesetzt werden kann. Dies ist insbesondere auf ihre hohe spezifische Aktivität sowie ihre hohe radioisotopische sowie chemische Reinheit zurückzuführen.

Mit dem erfindungsgemäßen Verfahren konnte die Routineproduktion von n.c.a. ¹⁷⁷Lu in industriellen Mengen erstmals etabliert werden.

Weitere Vorteile und Merkmale ergeben sich aufgrund der Beschreibung eines Ausführungsbeispiels sowie anhand der Zeichnung.

Es zeigt:
Fig. 1 einen schematischen Aufbau einer beispielhaften Vorrichtung zur Durchführung des erfindungsgemäßen Verfahrens;
Fig. 2 ein Säulenchromatogramm der Trennung von ¹⁷⁷Lu und Ytterbium, aufgenommen am Ausgang der Säule S1 in Fig. 1;
Fig. 3 ein Säulenchromatogramm der weiteren Abtrennung von ¹⁷⁷Lu von Ytterbium, aufgenommen am Ausgang der Säule S2 in Fig. 1; und
Fig. 4 ein SF-ICP-Massenspektrum des erfindungsgemäß erhaltenen trägerfreien ¹⁷⁷Lu-Endproduktes (n.c.a. ¹⁷⁷Lu) im Vergleich zu c.a. ¹⁷⁷Lu gemäß dem Stand der Technik.

Im Folgenden wird der beispielhafte Aufbau einer Vorrichtung zur Durchführung des erfindungsgemäßen Verfahrens unter Bezugnahme auf Fig. 1 beschrieben:
Der Prozess wird aus Strahlenschutzgründen in einer mit Blei und/oder Plexiglas abgeschirmten Umgebung durchgeführt. Dies kann eine Heiße Zelle oder ein anderes geeignetes System sein. Vor dem Hintergrund der Nutzung des Produktes als pharmazeutischen Wirkstoff, ist die Umgebung entsprechenden Reinheitsklassen nach den Anforderungen der pharmazeutischen Herstellung (good manufacturing practice, GMP der EU) einzustufen. In diesem Fall muss die Umgebungsbedingung in der Heißen Zelle Klasse C oder besser sein.

Die Heiße Zelle verfügt über geeignete Schleusensysteme zur Umgebung. Dort sind die Hilfssysteme für die Produktion wie HPLC-Pumpen, Spritzenpumpen oder anderer Fördersysteme sowie die Steuerung untergebracht.

Das System verfügt über mehrere Einzelkomponenten wie chromatographische Säulen (VS1, S1, VS2, S2 und S3), Flaschen (F1 bis F6) und Pumpen (P1 bis P7) die über Kapillaren und Ventile miteinander verbunden sind.

Die Pumpen können je nach Funktion als Vakuumpumpen, Spritzenpumpen, HPLC-Pumpen, Peristaltikpumpen oder anderen Wirkprinzipien ausgeführt sein. Im vorliegenden Ausführungsbeispiel sind die Pumpen (P1) und (P2) als HPLC-Pumpen ausgeführt. Sie fördern H₂O, HIBA und NH₄Cl in unterschiedlichen Konzentrationen (von 0,01 M bis 10 M) und Flussraten (von 0,05 ml/min bis 100 ml/min). Die Pumpen (P3), (P4), (P5), (P6) fördern weitere Reagenzien wie HCl, HNO₃, H₂O und Luft in unterschiedlichen Konzentrationen. (von 0,01 M bis 10 M) und Flussraten (von 0,05 ml/min bis 100 ml/min). Die Pumpen P3 bis P6 sind in der bevorzugten Ausführung Spritzen- oder Kolbenpumpen. Sie können aber auch durch weitere Ventile zu einem Pumpensystem in der Ausführung einer Spritzenpumpe realisiert werden. Die Pumpe 7 (P7) ist eine Vakuumpumpe, um einen variablen Unterdruck (von 1 mbar bis 1000 mbar) an das System anlegen zu können.

Die mit (N2) gekennzeichneten Komponenten (ohne eigene Nummerierung) sind Inertgasquellen, vorzugsweise Stickstoff oder Argon, die das System mit Druck zwischen 0,1 bar und bis zu 5 bar oder auch höher, je nach Auslegung des Systems beaufschlagen können.

Die Komponente (1) ist zum Aufbrechen von Ampullen und zusätzlich zur Konversion eines Ytterbiumoxids in Ytterbiumnitrat ausgeführt. Beide separaten Funktionen sind in diesem Beispiel in Funktionsintegration ausgeführt.

Die Komponente (2) ist eine Verdampfungseinheit zum Eintrocknen einer Lutetium-Lösung. Die Komponente (3) ist ein System zum Aufnehmen des finalen Produktes, beispielsweise ein Glasvial. Die Komponenten (2) und (3) können im Rahmen einer Funktionsintegration als ein Bauteil ausgeführt werden.

Sämtliche Ventile sind in diesem Ausführungsbeispiel so dargestellt, dass sie in jede Richtung schaltbar sind. Die Position der Ventile ist so gewählt, dass deren Anzahl minimiert ist. Wie für den Durchschnittsfachmann aus Fig. 1 ersichtlich ist, sind durchaus andere Ventilanordnungen denkbar, insbesondere zum Zusammenführen oder Trennen von Funktionen.

Die Flaschen (F1), (F2), (F3), (F4), (F5), (F6) sind Behälter zum Auffangen von Lösungen. Die bevorzugte Ausführungsform sind Glasflaschen mit einem für das erfindungsgemäße Verfahren angepassten Volumen. Insbesondere für größere Volumina ist die bevorzugte Ausführungsform ein Kunststoffkanister.

Das hier in der bevorzugt gezeigten Ausführungsform beispielhaft gezeigte Säulensystem umfasst sogenannte Vorsäulen (VS 1) und (VS2), durch die die Beladung vorgenommen wird. Die Hauptsäulen (S1) und (S2), die im Beispielsfalle die eigentlichen Trennsäulen bilden, schließen an die Vorsäulen and, so dass die jeweiligen Säulenpartner (VS1) und (S1) oder (VS2) und (S2) zu einem Säulensystem zusammengeschlossen werden können.

Das gesamte Fluidschema der beispielhaften Vorrichtung zur Durchführung der Erfindung ist unabhängig von der realen Anordnung - auch von der Anordnung innerhalb von Heißen Zellen - in Fig. 1 dargestellt. Eine bevorzugte Ausführungsform ist die Anordnung der Komponenten (2) und (3) in einer separaten abgeschirmten Einrichtung, um den Folgeprozess, das Abfüllen der für den Kunden vorgesehenen Mengen an ¹⁷⁷Lu in insgesamt einer Einrichtung zu ermöglichen. Sinnvollerweise sind die Komponenten (2) und (3) in ein System integriert. Eine weitere bevorzugte Ausführungsform ist die Anordnung der Komponente (3) in einer separaten abgeschirmten Einheit, so dass der vollständige Prozess in einer Einheit stattfindet und nur das Vial (3) zum Auffangen des Produktes in einer pharmazeutisch anspruchsvolleren Umgebung positioniert ist.

Zur Prozesskontrolle werden in diesem Beispiel Aktivitätssensoren eingesetzt, die jeweils am Ende der Säulen (S1), (S2) und (S3) positioniert sind, um den Verlauf der Trennung zu überwachen.

### Ausführungsbeispiel

Bei der vorliegenden Erfindung handelt es sich um einen Herstellungsprozess, bei dem ¹⁷⁷Lu n.c.a. aus Reaktorbestrahltem ¹⁷⁶Yb extrahiert wird. Dazu wird die bestrahlte Ampulle in einem Ampullenbecher geöffnet und in ein Konversionsgefäß (F1) überführt. Das ¹⁷⁶Yb kann als unlösliches Oxid vorliegen. Für die Extraktion des während der Bestrahlung entstandenen ¹⁷⁷Lu muss das Ausgangsmaterial in eine lösliche Form überführt werden. Dies kann im vorliegenden Ausführungsbeispiel durch den Einsatz von 1 M bis 12 M HNO₃ - bei Bedarf unter Erwärmung - erreicht werden.

Durch Verdünnung auf eine niedrigere Säurenkonzentration zwischen 0,01 M und 1,5 M HNO₃ kann die Lösung auf ein Vorsäulensystem (VS1) als erste Säule geladen werden. Das Säulenmaterial - ein makroporöser Kationenaustauscher auf Polystyrolbasis - des Vorsäulensystems wird durch die Beladung in eine für die Abtrennung negative H⁺ - Form (protonierte Form) überführt. Durch den Einsatz von NH₄Cl wird das Säulenmaterial des Vorsäulensystems in seine NH₄⁺ Form überführt. Anschließend wird das Vorsäulensystem VS1 mit Wasser gespült und mit der Trennsäule S1 als zweiter Säule verbunden.

Die Trennung wird mittels der Pumpe P1 bei hohen Flussraten (10-50 ml/min) durchgeführt. Dazu wird ein für die Trennung im VS1/S1 System optimierter Gradient aus Wasser und dem im Beispielsfalle als Komplexbildner eingesetzten α-HydroxyIsobutyrat (HIBA) ausgehend von 100% H₂O bis 0,2 M HIBA eingestellt und die Trennung durch das Vorsäulensystem VS1 und die Trennsäule S1 hindurch gefahren. Die Trennung wird mittels Dosierleistungssensoren überwacht. Sobald das ¹⁷⁷Lu von der Säule S1 eluiert wird, wird das Eluat in der Sammelflasche F2 aufgefangen.

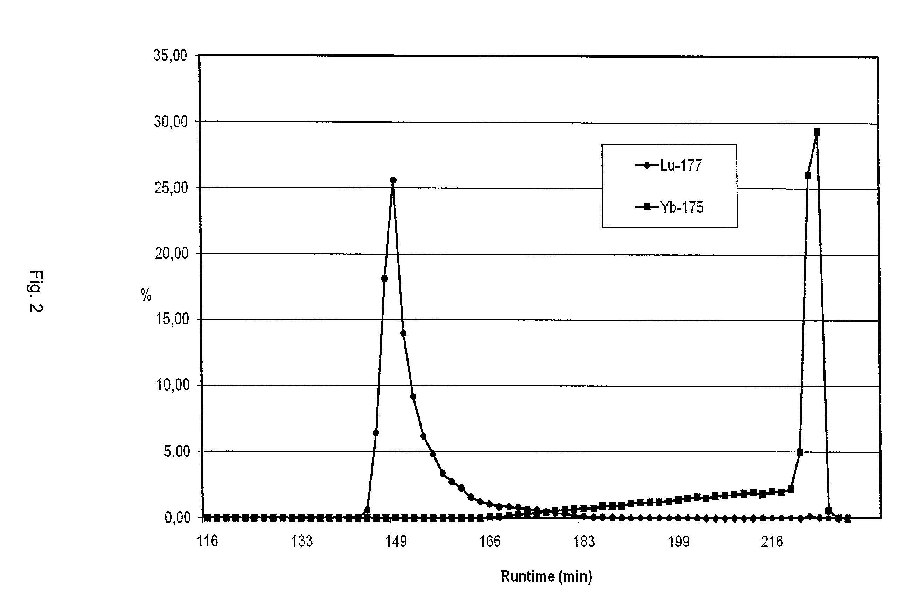

Die Trennung zwischen ¹⁷⁷Lu und Ytterbium ist in Fig. 2 als Chromatogramm gezeigt. Die Ordinate gibt die eluierte Menge des auf die Säule aufgegebenen ¹⁷⁷Lu bzw. Ytterbium in % an, die Abszisse gibt die Retentionszeit in Minuten an. Der massive Peak-Anstieg beim Ytterbium ist darauf zurück zu führen, dass kurz nach dem Maximum des Lutetium-Peaks auf eine hohe HIBA-Konzentration umgeschaltet wurde, damit das Ytterbium innerhalb einer vernünftigen Zeit und in einem akzeptablen Volumen erhalten werden kann.

Der im Eluat aus der Säule S1 noch enthaltende Komplexbildner - im Beispielsfalle HIBA - wird durch die Zugabe von Säure protoniert und damit wirkungslos gemacht. Nachdem das ¹⁷⁷Lu gesammelt ist, wird das Ytterbium durch den Einsatz von höher konzentrierter HIBA von der ersten und zweiten Säule eluiert und zum Zweck der Widerverwendung gesondert gesammelt.

Durch die Säurezugabe in die F2 kann das Eluat der S1 in einem zweiten Vorsäulensystem VS2 reteniert werden. Das Eluat wird im Beispielsfalle durch Stickstoffdruck, noch während weiteres Eluat gesammelt wird, auf das Vorsäulensystem VS2 als dritte Säule aufgetragen. Dabei ist in regelmäßigen Abständen oder auch kontinuierlich eine Säurezugabe in die Flasche F2 erforderlich. Das Säulenmaterial des Systems VS2 wird bei der Beladung ebenfalls in seine H⁺ Form überführt. Für die Überführung der unerwünschten H⁺ Form in die für die Trennung bevorzugte NH₄⁺ Form wird das VS2 System mit NH₄Cl und anschließend mit Wasser gespült. Anschließend wird das Vorsäulensystem VS2 mit der Trennsäule S2 als vierter Säule verbunden.

Die weitere Trennung wird mittels einer HPLC Pumpe P2 bei mittleren Flussraten (1-10 ml/min) durchgeführt. Dazu wird ein auf die Trennung im VS2/S2 System optimierter Gradient aus Wasser und HIBA wie oben erwähnt eingestellt und die Trennung durch das Vorsäulensystem VS2 und die Trennsäule S2 hindurch gefahren.

Die Trennung wird mittels Dosisleistungssensoren überwacht. Sobald das ¹⁷⁷Lu von der Säule S2 eluiert wird, wird das Eluat in der Sammelflasche F3 aufgefangen. Der im Eluat noch enthaltende Komplexbildner HIBA wird durch die Zugabe von Säure protoniert und damit wirkungslos gemacht. Nachdem das ¹⁷⁷Lu gesammelt ist, wird das Ytterbium durch den Einsatz von höher konzentrierter HIBA von den Säulen VS2 und S2 eluiert und zum Zweck der Wiederverwendung gesondert gesammelt.

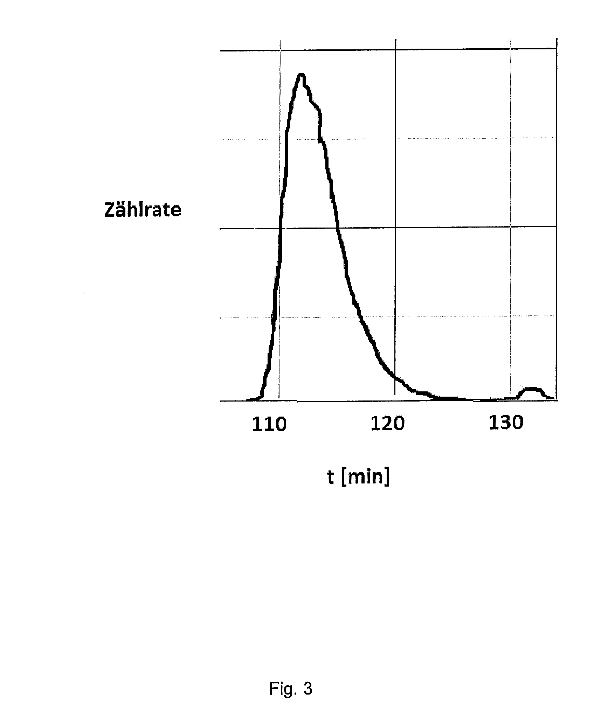

Fig. 3 zeigt einen Ausschnitt aus einem Säulenchromatogramm an Säule S2, bei welchem wiederum die Dosisleistung (Dose rate) gegen die Retentionszeit in Minuten aufgetragen ist. Ähnlich wie in Fig. 2 erscheint in Fig. 3 der (jetzt nur noch sehr kleine) Ytterbium-Peak nur deswegen so kurz (bei einer Retentionszeit von ca. 135) nach dem Lutetium-Peak, da kurz nach dem Maximum des Lutetium-Peaks (ca. 115 min) auf eine hohe HIBA-Konzentration umgeschaltet wurde. Ansonsten würde das Ytterbium in dieser Trennung erst nach mehreren Stunden auftauchen, was den Prozess unnötig verzögern würde, da es natürlich sinnvoll ist, das Ytterbium, insbesondere ¹⁷⁶Yb, einer Wiederverwendung zuzuführen.

Das Eluat der Säule S2 wird aus der Sammelflasche F3 auf eine finale Säule S3 als fünfter Säule geladen. Dazu wird das Eluat noch während des Sammelns durch Stickstoffdruck aus der Sammelflasche F3 auf die Säule S3 aufgetragen. Dabei ist in regelmäßigen Abständen eine Säurezugabe in die Flasche F3 erforderlich. Nach Abschluss der Beladung der finalen Trennsäule S3 wird diese durch Spülen mit verdünnter Säure von HIBA befreit. Das selektive Spülen der Säule S3 mit Säure unterschiedlicher Konzentrationen ermöglicht eine weitere Abtrennung von Fremdmetallspuren bzw. -verunreinigungen.

Nach der finalen Aufreinigung auf der Säule S3 wird das ¹⁷⁷Lu mittels hoch konzentrierter Säure in eine Verdampfungseinheit 2 eluiert. Die Säure wird durch Verdampfung entfernt. Dieser Schritt dient auch zur gleichzeitigen Sterilisation des Endproduktes.

Das ¹⁷⁷Lu n.c.a. kann nun in dem gewünschten Lösungsmittel und in der erwünschten Konzentration aufgenommen werden. Nach einer finalen Bestimmung der erhaltenden Aktivität und einer Qualitätsüberprüfung wird das hergestellte ¹⁷⁷Lu den Kundenwünschen entsprechend in ein Vial 3 abgefüllt.

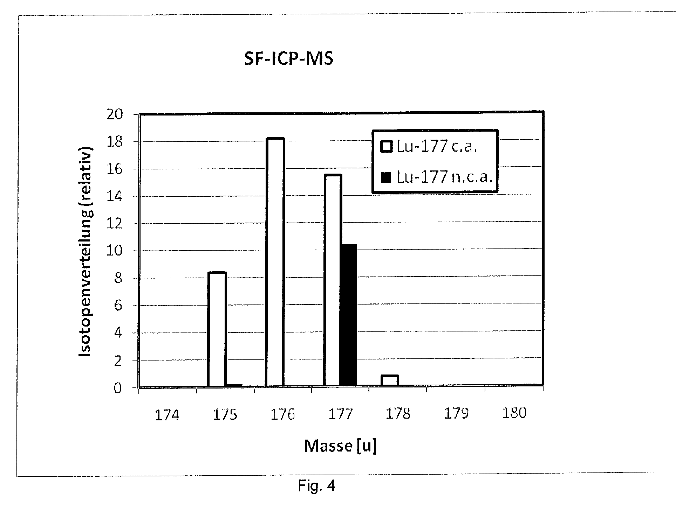

Typischerweise ist die mittels des vorliegenden Verfahrens erhaltene trägerfreie ¹⁷⁷Lu-Verbindung dadurch gekennzeichnet, dass sie im SF-ICP-Massenspektrum lediglich einen Peak bei einer Atommasse von 177 aufweist, während c.a. ¹⁷⁷ Lu im Wesentlichen drei Hauptpeaks bei 175, 176 und 177 Atommasseeinheiten aufweist. Dieser Unterschied ist in dem Massenspektrum der Fig. 4 gezeigt. Die Ordinate gibt die Isotopenverteilung auf einer relativen Häufigkeitsskala von 0 bis 12 an. Die Abszisse in Fig. 4 gibt die Atommasse an. Als massenspektroskopisches Verfahren kam eine Sektorfeldmassenspektrometrie mit induktiv gekoppeltem Plasma [Sector Field Inductively Coupled Plasma - Mass Spectrometry, SF-ICP-MS] zum Einsatz.

## Patentansprüche

1. Verfahren zur Herstellung im Wesentlichen trägerfreier hochreiner ¹⁷⁷Lu-Verbindungen für medizinische Zwecke aus mit thermischen Neutronen bestrahlten ¹⁷⁶Yb-Verbindungen, wobei die Endprodukte der Neutronenbestrahlung, die im Wesentlichen eine Mischung aus die ¹⁷⁷Lu und ¹⁷⁶Yb im Massenverhältnis von ca. 1:10² bis 1:10¹⁰ enthalten, als Ausgangsmaterialien eingesetzt werden, wobei in Wasser unlösliche Ausgangsmaterialien mittels Mineralsäuren in eine lösliche Form überführt werden, und wobei das Verfahren folgende Schritte umfasst:
a) Beladen einer ersten Säule (VS1), die mit einem Kationenaustauschermaterial gepackt ist, mit den in Mineralsäure gelösten Ausgangsmaterialien, die ¹⁷⁷Lu und ¹⁷⁶Yb im Massenverhältnis von ca. 1:10² bis 1:10¹⁰ enthalten; Austauschen der Protonen des Kationenaustauschermateriales gegen Ammoniumionen unter Einsatz einer NH₄Cl-Lösung; und Spülen des Kationenaustauschermateriales der ersten Säule (VS1) mit Wasser;
b) Verbinden des Ausgangs der ersten Säule (VS1) mit dem Eingang einer zweiten Säule (S1), die ebenfalls mit einem Kationenaustauschermaterial gepackt ist;
c) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner an den Eingang der ersten Säule (VS1), um ¹⁷⁷Lu-Verbindungen von der ersten (VS1) und der zweiten Säule (S1) zu eluieren;
d) Erfassen der Radioaktivitätsdosis am Ausgang der zweiten Säule (S1), um eine Elution von ¹⁷⁷Lu-Verbindungen zu erkennen; und Auffangen eines ersten ¹⁷⁷Lu-Eluates vom Ausgang der zweiten Säule (S1) in einem Gefäß (F2); und Protonieren des Komplexbildners, um diesen für die Komplexbildung mit ¹⁷⁷Lu-Ionen unwirksam zu machen;
e) Beladung einer finalen Trennsäule (S3), die mit einem Kationenaustauschermaterial gepackt ist, durch kontinuierliche Weiterleitung des sauren ¹⁷⁷Lu-Eluates aus Schritt d) zum Eingang der finalen Trennsäule (S3); Auswaschen des Komplexbildners mit verdünnter Mineralsäure einer Konzentration von kleiner als ca. 0,1 M; Entfernen von Fremdmetallionenspuren aus der ¹⁷⁷Lu-Lösung durch Waschen des Kationenaustauschermateriales der finalen Trennsäule (S3) mit Mineralsäure unterschiedlicher Konzentrationen im Bereich von ca. 0,1 bis 2,5 M;
f) Eluieren der ¹⁷⁷Lu-Ionen von der finalen Trennsäule (S3) mittels einer hochkonzentrierten Mineralsäure von ca. 3 bis 12 M; Auffangen des hochreinen ¹⁷⁷Lu-Eluates in einer Verdampfereinrichtung und Entfernen der Mineralsäure durch Verdampfen.

2. Verfahren nach Anspruch 1, **dadurch gekennzeichnet, dass** zwischen den Schritten d) und f) die folgenden Schritte zusätzlich durchgeführt werden:
d.1) kontinuierliche Weiterleitung des sauren ¹⁷⁷Lu-Eluates aus Schritt d) zum Eingang einer dritten Säule (VS2), die mit einem Kationenaustauschermaterial gepackt ist, wobei das Kationenaustauschermaterial durch die Beladung mit dem sauren ¹⁷⁷Lu-Eluat in protonierter Form vorliegt; Austauschen der Protonen des Kationenaustauschermateriales gegen Ammoniumionen unter Einsatz einer NH₄Cl-Lösung; und Spülen des Kationenaustauschermateriales der dritten Säule (VS2) mit Wasser;
d.2) Verbinden des Ausgangs der dritten Säule (VS2) mit dem Eingang einer vierten Säule (S2), die mit einem Kationenaustauschermaterial gepackt ist;
d.3) Anlegen eines Gradienten aus Wasser und einemm Komplexbildner ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner an den Eingang der dritten Säule (VS2), um ¹⁷⁷Lu-Verbindungen von der dritten (VS2) und der vierten Säule (S2) zu eluieren;
d.4) Erfassen der Radioaktivitätsdosis am Ausgang der vierten Säule (S2), um eine Elution der ¹⁷⁷Lu-Verbindungen zu erkennen; und Auffangen eines zweiten ¹⁷⁷Lu-Eluates vom Ausgang der dritten Säule (S2) in einem Gefäß (F3); und Protonieren des Komplexbildners, um diesen für die Komplexbildung mit ¹⁷⁷Lu-Ionen unwirksam zu machen.

3. Verfahren nach Anspruch 1 oder 2, **dadurch gekennzeichnet, dass** nach der Elution der ¹⁷⁷Lu-Verbindungen in den Schritten und d) und d.4) die erste (VS1) und zweite (S1) Säule sowie die dritte (VS2) und die vierte (S2) Säule mit höheren Komplexbildnerkonzentrationen gewaschen werden, um Yb-Ionen von dem Kationenaustauschermaterial zu eluieren; und erhaltene Yb-Eluate, welche im Wesentlichen ¹⁷⁶Yb-Ionen enthalten, zum Zwecke der Wiederverwendung als Ausgangsmaterial für die ¹⁷⁷Lu-Herstellung gesondert gesammelt werden.

4. Verfahren nach einem der vorhergehenden Ansprüche, **dadurch gekennzeichnet, dass** als Säure HNO₃ , HCl, HF oder H₂SO₄ oder organische Säuren, insbesondere Essigsäure, verwendet wird.

5. Verfahren nach einem der vorhergehenden Ansprüche, **dadurch gekennzeichnet, dass** in Wasser unlösliche ¹⁷⁶Yb-Oxide durch den Einsatz von 1M bis 12M HNO₃, H₂SO₄ oder andere oxidierende Säuren in eine wasserlösliche Form überführt werden.

6. Verfahren nach einem der vorhergehenden Ansprüche, **dadurch gekennzeichnet, dass** die Beladung der Kationenaustauschermaterialien mit einer Säurekonzentration von 0,01 M bis 2 M HNO₃, HCl, oder anderen anorganischen und/oder organischen Säuren erfolgt.

7. Verfahren nach einem der vorhergehenden Ansprüche, **dadurch gekennzeichnet, dass** das Kationenaustauschermaterial ausgewählt wird aus der Gruppe bestehend aus: makroporösen und gelartigen Kationenaustauschharzen auf Basis organischer Polymere, insbesondere solchen auf Polystyrolbasis; und Kationenaustauschharzen auf Silikatbasis.

8. Verfahren nach einem der vorhergehenden Ansprüche, **dadurch gekennzeichnet, dass** Grammmengen an Ausgangsmaterialien eingesetzt werden und Milligrammmengen an ¹⁷⁷Lu produziert werden.

9. Verfahren nach einem der vorhergehenden Ansprüche, **dadurch gekennzeichnet, dass** Ausbeuten von mehren TBq ¹⁷⁷Lu und spezifische Aktivitäten von ca. 3,9 TBq ¹⁷⁷Lu pro mg Lutetium erhalten werden.

10. Verfahren zur Herstellung im Wesentlichen trägerfreier hochreiner ¹⁷⁷Lu-Verbindungen für medizinische und/oder diagnostische Zwecke aus mit thermischen Neutronen bestrahlten ¹⁷⁶Yb-Verbindungen, wobei die Endprodukte der Neutronenbestrahlung, die im Wesentlichen eine Mischung aus die ¹⁷⁷Lu und ¹⁷⁶Yb im Massenverhältnis von ca. 1:10² bis 1:10¹⁰ enthalten, als Ausgangsmaterialien eingesetzt werden, wobei in Wasser unlösliche Ausgangsmaterialien mittels Mineralsäuren in eine lösliche Form überführt werden, und wobei das Verfahren folgende Schritte umfasst:
a) Beladen einer ersten Säule (VS1), die mit einem Kationenaustauschermaterial gepackt ist, mit den in Mineralsäure gelösten Ausgangsmaterialien, die ¹⁷⁷Lu und ¹⁷⁶Yb im Massenverhältnis von ca. 1:10² bis 1:10¹⁰ enthalten; Austauschen der Protonen des Kationenaustauschermateriales gegen Ammoniumionen unter Einsatz einer NH₄Cl-Lösung; und Spülen des Kationenaustauschermateriales der ersten Säule (VS1) mit Wasser;
b) Verbinden des Ausgangs der ersten Säule (VS1) mit dem Eingang einer zweiten Säule (S1), die ebenfalls mit einem Kationenaustauschermaterial gepackt ist;
c) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner an den Eingang der ersten Säule (VS1);
d) Erfassen der Radioaktivitätsdosis am Ausgang der zweiten Säule (S1), um eine Elution von ¹⁷⁷Lu-Verbindungen zu erkennen; und Auffangen eines ersten ¹⁷⁷Lu-Eluates vom Ausgang der zweiten Säule (S1) in einem Gefäß (F2); und Protonieren des Komplexbildners, um diesen für die Komplexbildung mit ¹⁷⁷Lu-Ionen unwirksam zu machen;
e) kontinuierliche Weiterleitung des sauren ¹⁷⁷Lu-Eluates aus Schritt d) zum Eingang einer dritten Säule (VS2), die mit einem Kationenaustauschermaterial gepackt ist, wobei das Kationenaustauschermaterial durch die Beladung mit dem sauren ¹⁷⁷Lu-Eluat in protonierter Form vorliegt; Austauschen der Protonen des Kationenaustauschermateriales gegen Ammoniumionen unter Einsatz einer NH₄Cl-Lösung; und Spülen des Kationenaustauschermateriales der dritten Säule (VS2) mit Wasser;
f) Verbinden des Ausgangs der dritten Säule (VS2) mit dem Eingang einer vierten Säule (S2), die mit einem Kationenaustauschermaterial gepackt ist;
g) Anlegen eines Gradienten aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner, an den Eingang der dritten Säule (VS2);
h) Erfassen der Radioaktivitätsdosis am Ausgang der vierten Säule (S2), um eine Elution der ¹⁷⁷Lu-Verbindungen zu erkennen; und Auffangen eines zweiten ¹⁷⁷Lu-Eluates vom Ausgang der dritten Säule (S2) in einem Gefäß (F3); und Protonieren des Komplexbildners, um diesen für die Komplexbildung mit ¹⁷⁷Lu-Ionen unwirksam zu machen;
i) Beladung einer fünften Säule (S3), die mit einem Kationenaustauschermaterial gepackt ist, durch kontinuierliche Weiterleitung des sauren ¹⁷⁷Lu-Eluates aus Schritt h) zum Eingang der fünften Säule (S3); Auswaschen des Komplexbildners mit verdünnter Mineralsäure; Entfernen von Fremdmetallionenspuren aus der ¹⁷⁷Lu-Lösung durch Waschen des Kationenaustauschermateriales der fünften Säule (S3) mit Mineralsäure unterschiedlicher Konzentrationen im Bereich von 0,01 bis 2,5 M;
j) Eluieren der ¹⁷⁷Lu-Ionen von der fünften Säule mittels einer konzentrierten Mineralsäure von ca. 1 M bis hin zu ca. 12 M; Auffangen des hochreinen ¹⁷⁷Lu-Eluates in einer Verdampfereinrichtung und Entfernen der Mineralsäure durch Verdampfen.

11. Verfahren nach einem der vorhergehenden Ansprüche, **dadurch gekennzeichnet, dass** es in einer heißen Zelle der Klasse C gemäß den GMP-Richtlinien der EU durchgeführt wird.

12. Verwendung einer Kationenaustauschchromatographie-Säule mit einem Kationenaustauscherharz als stationäre Phase und einem Komplexbildner als mobile Phase zum Herstellen von hochreinen, trägerfreien ¹⁷⁷Lu-Verbindungen im Milligrammmaßstab aus einer ¹⁷⁶Yb-Matrix im Grammmaßstab, wobei die Säule mit einem Kationenaustauschermaterial gepackt ist, und die Protonen des Kationenaustauschermateriales gegen Ammoniumionen unter Einsatz einer NH4 Cl-Lösung ausgetauscht wurden;
ein Gradient aus Wasser und einem Komplexbildner, ausgewählt aus der Gruppe bestehend aus: α-Hydroxyisobutyrat [HIBA], Zitronensäure, Zitrat, Buttersäure, Butyrat, EDTA, EGTA und Ammoniumionen, beginnend bei 100% H₂O bis 0,2 M Komplexbildner, an den Eingang der Säule angelegt wird; und eine Radioaktivitätsdosis am Ausgang der Säule erfasst wird, um eine Elution der ¹⁷⁷Lu-Verbindungen zu erkennen.

## Claims

1. Method of manufacturing essentially non-carrier-added high-purity ¹⁷⁷Lu compounds for medicinal purposes from ¹⁷⁶Yb compounds irradiated with thermal neutrons, wherein the end products of neutron irradiation, which essentially contain a mixture of ¹⁷⁷Lu and ¹⁷⁶Yb in an approximate mass ratio of 1:10² to 1:10¹⁰, are used as base materials, wherein base materials that are insoluble in water are converted into a soluble form by way of mineral acids, and wherein the method comprises the following steps:
a) loading a first column (VS1) packed with cation exchange material, with the base materials solved in mineral acid and containing ¹⁷⁷Lu and ¹⁷⁶Yb in an approximate mass ratio of 1:10² to 1:10¹⁰ ; exchanging the protons of the cation exchange material for ammonium ions, thereby using an NH₄Cl solution; and washing the cation exchange material of the first column (VS1) with water;
b) linking the outlet of the first column (VS1) with the inlet of a second column (S1) that likewise is packed with a cation exchange material;
c) applying a gradient of water and a chelating agent selected from the group consisting of: α-hydroxyisobutyrate [HIBA], citric acid, citrate, butyric acid, butyrate, EDTA, EGTA and ammonium ions, starting at 100% of H₂0 to 0.2 M of the chelating agent on the inlet of the first column (VS1), so as to elute ¹⁷⁷Lu compounds from the first (VS1) and second column (S1);
d) determining the radioactivity dose at the outlet of the second column (S1) in order to recognize the elution of ¹⁷⁷Lu compounds; and collecting a first ¹⁷⁷Lu eluate from the outlet of the second column (S1) in a vessel (F2); and protonating the chelating agent so as to inactivate the same for the complex formation with ¹⁷⁷Lu ions;
e) loading a final separation column (S3) packed with a cation exchange material by continuously conveying the acidic ¹⁷⁷Lu eluate of step d) to the inlet of the final separation column (S3); washing out the chelating agent with diluted mineral acid of a concentration lower than approximately 0.1 M; removing traces of other metal ions from the ¹⁷⁷Lu solution by washing the cation exchange material of the final separation column (S3) with mineral acid of various concentrations in a range of approximately 0.1 to 2.5 M;
f) eluting the ¹⁷⁷Lu ions from the final separation column (S3) by way of a highly concentrated mineral acid of approximately 3 to 12 M; collecting the high purity ¹⁷⁷Lu eluate in a vaporizer unit and removing the mineral acid by vaporization.

2. Method in accordance with claim 1, **characterized in that** between steps d) and f) the following steps are performed additionally:
d.1) continuously conveying the acidic ¹⁷⁷Lu eluate of step d) to the inlet of a third column (VS2) packed with cation exchange material, the cation exchange material being present in protonated form due to the loading with acidic ¹⁷⁷Lu eluate; exchanging the protons of the cation exchange material for ammonium ions, thereby using an NH₄Cl solution; and washing the cation exchange material of the third column (VS2) with water;
d.2) linking the outlet of the third column (VS2) with the inlet of a fourth column (S2) packed with a cation exchange material;
d.3) applying a gradient of water and a chelating agent selected from the group consisting of: α-hydroxyisobutyrate [HIBA], citric acid, citrate, butyric acid, butyrate, EDTA, EGTA and ammonium ions, starting at 100% of H₂0 to 0.2 M of the chelating agent, on the inlet of the third column (VS2) so as to elute ¹⁷⁷Lu compounds from the third (VS2) and fourth column (S2);
d.4) determining the radioactivity dose at the outlet of the fourth column (S2) in order to recognize the elution of ¹⁷⁷Lu compounds; and collecting a second ¹⁷⁷Lu eluate from the outlet of the third column (S2) in a vessel (F3); and protonating the chelating agent so as to inactivate the same for the complex formation with ¹⁷⁷Lu ions.

3. The method in accordance with claims 1 or 2, **characterized in that** after elution of the ¹⁷⁷Lu compounds in steps d) and d.4), the first (VS1) and second (S1) column as well as the third (VS2) and fourth (S2) column are washed using higher concentrations of chelating agents so as to elute Yb ions from the cation exchange material; and Yb eluates obtained that essentially contain ¹⁷⁶Yb ions, are collected separately for the purpose of re-using them as base material for the manufacture of ¹⁷⁷Lu.

4. The method in accordance with any of the preceding claims, **characterized in that** HNO₃, HCl, HF or H₂SO₄ or organic acids, in particular acetic acid, are used as acid.

5. The method in accordance with any of the preceding claims, **characterized in that** ¹⁷⁶Yb oxides insoluble in water are converted into a water-soluble form by the use of 1 M to 12 M of HNO₃, H₂SO₄ or other oxidizing acids.

6. The method in accordance with any of the preceding claims, **characterized in that** loading of the cation exchange materials is done using an acid concentration of 0.01 M to 2 M of HNO₃, HCl or other inorganic and/or organic acids.

7. The method in accordance with any of the preceding claims, **characterized in that** the cation exchange material is selected from the group consisting of: macroporous and gel-like cation exchange resins on the basis of organic polymers, in particular those on a polystyrene basis; and cation exchange resins on a silicate basis.

8. The method in accordance with any of the preceding claims, **characterized in that** gram amounts of base materials are used and milligram amounts of ¹⁷⁷Lu are produced.

9. The method in accordance with any of the preceding claims, **characterized in that** yields of several TBq of ¹⁷⁷Lu and specific activities of approximately 3.9 TBq of ¹⁷⁷Lu per mg of lutetium are obtained.

10. Method of manufacturing essentially non-carrier-added high purity ¹⁷⁷Lu compounds for medicinal and/or diagnostic purposes from ¹⁷⁶Yb compounds irradiated with thermal neutrons, wherein the end products of neutron irradiation, which essentially contain a mixture of ¹⁷⁷Lu and ¹⁷⁶Yb in an approximate mass ratio of 1:10² to 1:10¹⁰, are used as base materials, wherein base materials that are insoluble in water, are converted into a soluble form by way of mineral acids, and wherein the method comprises the following steps:
a) loading a first column (VS1) packed with cation exchange material, with the base materials solved in mineral acid and containing ¹⁷⁷Lu and ¹⁷⁶Yb in an approximate mass ratio of 1:10² to 1:10¹⁰; exchanging the protons of the cation exchange material for ammonium ions, thereby using an NH₄Cl solution; and washing the cation exchange material of the first column (VS1) with water;
b) linking the outlet of the first column (VS1) with the inlet of a second column (S1) that likewise is packed with a cation exchange material;
c) applying a gradient of water and a chelating agent selected from the group consisting of: α-hydroxyisobutyrate [HIBA], citric acid, citrate, butyric acid, butyrate, EDTA, EGTA and ammonium ions, starting at 100% of H₂0 to 0.2 M of the chelating agent on the inlet of the first column (VS1);
d) determining the radioactivity dose at the outlet of the second column (S1) in order to recognize the elution of ¹⁷⁷Lu compounds; and collecting a first ¹⁷⁷Lu eluate from the outlet of the second column (S1) in a vessel (F2); and protonating the chelating agent so as to inactivate the same for the complex formation with ¹⁷⁷Lu ions;
e) continuously conveying the acidic ¹⁷⁷Lu eluate of step d) to the inlet of a third column (VS2) packed with cation exchange material, the cation exchange material being present in protonated form due to the loading with acidic ¹⁷⁷Lu eluate; exchanging the protons of the cation exchange material for ammonium ions, thereby using an NH₄Cl solution; and washing the cation exchange material of the third column (VS2) with water;
f) linking the outlet of the third column (VS2) with the inlet of a fourth column (S2) packed with a cation exchange material;
g) applying a gradient of water and a chelating agent selected from the group consisting of: α-hydroxyisobutyrate [HIBA], citric acid, citrate, butyric acid, butyrate, EDTA, EGTA and ammonium ions, starting at 100% of H₂0 to 0.2 M of the chelating agent, on the inlet of the third column (VS2);
h) determining the radioactivity dose at the outlet of the fourth column (S2) in order to recognize the elution of ¹⁷⁷Lu compounds; and collecting a second ¹⁷⁷Lu eluate from the outlet of the third column (S2) in a vessel (F3); and protonating the chelating agent so as to inactivate the same for the complex formation with ¹⁷⁷Lu ions;
i) loading a fifth column (S3) packed with cation exchange material by continuously conveying the acidic ¹⁷⁷Lu eluate of step h) to the inlet of the fifth column (S3); washing the chelating agent with diluted mineral acid; removing traces of other metal ions from the ¹⁷⁷Lu solution by washing the cation exchange material of the fifth column (S3) with mineral acid of various concentrations in a range of approximately 0.01 to 2.5 M;
j) eluting the ¹⁷⁷Lu ions from the fifth column by way of concentrated mineral acid of approximately 1 M up to approximately 12 M; collecting the high purity ¹⁷⁷Lu eluate in a vaporizer unit and removing the mineral acid by vaporization.

11. The method in accordance with any of the preceding claims, **characterized in that** it is performed in a hot cell of class C in accordance with EC GMP directives.

12. The use of a cation exchange chromatography column with a cation exchange resin as stationary phase and a chelating agent as mobile phase for the manufacture of high-purity, non-carrier-added ¹⁷⁷Lu compounds on a milligram scale from a ¹⁷⁶Yb matrix on a gram scale, wherein the column is packed with cation exchange material, and the protons of the cation exchange material were exchanged for ammonium ions using an NH₄Cl solution;
a gradient of water and a chelating agent selected from the group consisting of: α-hydroxyisobutyrate [HIBA], citric acid, citrate, butyric acid, butyrate, EDTA, EGTA and ammonium ions, starting at 100% of H₂0 to 0.2 M of the chelating agent, is applied on the inlet of the column; and
the radioactivity dose at the outlet of the column is determined in order to recognize the elution of ¹⁷⁷Lu compounds.

## Revendications

1. Procédé servant à fabriquer des composés ¹⁷⁷Lu à pureté élevée essentiellement sans porteur à des fins médicales à partir de composés ¹⁷⁶Yb irradiés de neutrons thermiques, sachant que les produits finaux de l'irradiation de neutrons, qui contiennent essentiellement un mélange composé des composés ¹⁷⁷Lu et ¹⁷⁶Yb selon un rapport massique allant d'environ 1:10² à 1:10¹⁰, sont utilisés en tant que matériaux de départ, sachant que des matériaux de départ insolubles dans l'eau sont transformés, au moyen d'acides minéraux, en une forme soluble, et sachant que le procédé comprend les étapes qui suivent consistant à :
a) charger une première colonne (VS1), qui est remplie d'un matériau échangeur de cations, avec les matériaux de départ dissous dans de l'acide minéral, lesquels contiennent les composés ¹⁷⁷Lu et ¹⁷⁶Yb selon un rapport massique allant d'environ 1:10² à 1:10¹⁰ ; remplacer les protons du matériau échangeur de cations contre des ions d'ammonium en utilisant une solution de NH₄Cl ; et rincer le matériau échangeur de cations de la première colonne (VS1) avec de l'eau ;
b) relier la sortie de la première colonne (VS1) à l'entrée d'une deuxième colonne (S1), qui est de la même manière remplie d'un matériau échangeur de cations ;
c) appliquer un gradient composé d'eau et d'un agent complexant, choisi DANS le groupe constitué de : α-hydroxybutyrate [HIBA], acide citrique, citrate, acide butyrique, butyrate, EDTA, EGTA et ions d'ammonium, en commençant à 100 % d'H₂O à 0,2 M d'un agent complexant à l'entrée de la première colonne (VS1) afin d'éluer des composés ¹⁷⁷Lu de la première (VS1) et de la deuxième (S1) colonne ;
d) détecter la dose de radioactivité à la sortie de la deuxième colonne (S1) afin d'identifier une élution de composés ¹⁷⁷Lu ; et recueillir un premier éluat ¹⁷⁷Lu provenant de la sortie de la deuxième colonne (S1) dans un récipient (F2) ; et protoner l'agent complexant afin de le rendre inactif pour la complexation avec des ions ¹⁷⁷Lu ;
e) charger une colonne de séparation finale (S3) qui est remplie, d'un matériau échangeur de cations, en acheminant en continu l'éluat ¹⁷⁷Lu acide obtenu à l'étape d) à l'entrée de la colonne de séparation finale (S3) ; laver l'agent complexant avec un acide minéral dilué d'une concentration inférieure à environ 0,1 M ; éliminer des traces d'ions métalliques étrangers de la solution ¹⁷⁷Lu en lavant le matériau échangeur de cations de la colonne de séparation finale (S3) avec un acide minéral à des concentrations différentes comprises dans la plage allant d'environ 0,1 à 2,5 M ;
f) éluer les ions ¹⁷⁷Lu de la colonne de séparation finale (S3) au moyen d'un acide minéral à concentration hautement élevée allant d'environ 3 à 12 M ; recueillir l'éluat ¹⁷⁷Lu à pureté élevée dans un dispositif d'évaporation et éliminer l'acide minéral par évaporation.

2. Procédé selon la revendication 1, **caractérisé en ce que** sont réalisées en supplément, entre les étapes d) et f), les étapes qui suivent consistant à :
d.1) acheminer en continu l'éluat ¹⁷⁷Lu acide obtenu à l'étape d) vers l'entrée d'une troisième colonne (VS2), qui est remplie d'un matériau échangeur de cations, sachant que le matériau échangeur de cations est présent sous une forme protonée du fait du chargement en éluat ¹⁷⁷Lu acide ; remplacer les protons du matériau échangeur de cations par des ions d'aluminium en utilisant une solution de NH₄Cl ; et rincer le matériau échangeur de cations de la troisième colonne (VS2) avec de l'eau ;
d.2) relier la sortie de la troisième colonne (VS2) à l'entrée d'une quatrième colonne (S2), qui est remplie d'un matériau échangeur de cations ;
d.3) appliquer un gradient composé d'eau et d'un agent complexant choisi parmi le groupe constitué de : α-hydroxybutyrate [HIBA], acide citrique, citrate, acide butyrique, butyrate, EDTA, EGTA et ions d'ammonium, en commençant à 100 % d'H₂O à 0,2 M d'agent complexant à l'entrée de la troisième colonne (VS2) afin d'éluer des composés ¹⁷⁷Lu de la troisième (VS2) et de la quatrième (S2) colonne ;
d.4) détecter la dose de radioactivité à la sortie de la quatrième colonne (S2) afin d'identifier une élution des composés ¹⁷⁷Lu ; et recueillir un deuxième éluat ¹⁷⁷Lu provenant de l'entrée de la troisième colonne (S2) dans un récipient (F3) ; et protoner l'agent complexant afin de le rendre inactif pour la complexation avec des ions ¹⁷⁷Lu.

3. Procédé selon la revendication 1 ou 2, **caractérisé en ce qu'**après l'élution des composés ¹⁷⁷Lu aux étapes d) et d.4), la première (VS1) et la deuxième (S1) colonne ainsi que la troisième (VS2) et la quatrième (S2) colonne sont lavées à des concentrations d'agent complexant plus élevées afin d'éluer des ions Yb du matériau échangeur de cations ; et collecter de manière séparée des éluats Yb obtenus, qui contiennent essentiellement des ions ¹⁷⁶Yb, à des fins de réutilisation en tant que matériau de départ pour la fabrication de composés ¹⁷⁷Lu.

4. Procédé selon l'une quelconque des revendications précédentes, **caractérisé en ce qu'**on utilise, en tant qu'acide, du HNO₃, du HCl, du HF ou du H₂SO₄ ou des acides organiques tels que de l'acide acétique.

5. Procédé selon l'une quelconque des revendications précédentes, **caractérisé en ce que** des oxydes ¹⁷⁶Yb insolubles dans l'eau sont transformés sous une forme hydrosoluble en utilisant 1 M à 12 M de HNO₃, de H₂SO₄ ou d'autres acides oxydants.

6. Procédé selon l'une quelconque des revendications précédentes, **caractérisé en ce que** le chargement des matériaux échangeurs de cations est effectué à une concentration d'acide allant de 0,01 M à 2 M de HNO₃, d'HCl ou d'autres acides inorganiques et/ou organiques.

7. Procédé selon l'une quelconque des revendications précédentes, **caractérisé en ce que** le matériau échangeur de cations est choisi parmi le groupe constitué de : résines échangeuses de cations macroporeuses et sous forme de gel à base de polymères organiques, en particulier de résines à base de polystyrène, et de résines échangeuses de cations à base de silicate.

8. Procédé selon l'une quelconque des revendications précédentes, **caractérisé en ce que** des quantités de l'ordre du gramme en matériaux de départ sont utilisées, et des quantités de l'ordre du milligramme en ¹⁷⁷Lu sont produites.

9. Procédé selon l'une quelconque des revendications précédentes, **caractérisé en ce que** sont obtenus des rendements de plusieurs TBq de composés ¹⁷⁷Lu et des activités spécifiques d'environ 3,9 TBq de composés ¹⁷⁷Lu par mg de lutétium.

10. Procédé servant à fabriquer des composés ¹⁷⁷Lu à pureté élevée essentiellement sans porteur à des fins médicales et/ou de diagnostic à partir de composés ¹⁷⁶Yb irradiés de neutrons thermiques, sachant que les produits finaux de l'irradiation de neutrons, qui contiennent essentiellement un mélange composé des composés ¹⁷⁷Lu et ¹⁷⁶Yb selon un rapport massique allant d'environ 1:10² à 1:10¹⁰, sont utilisés en tant que matériaux de départ, sachant que des matériaux de départ insolubles dans l'eau sont transformés sous une forme soluble au moyen d'acides minéraux et sachant que le procédé comprend les étapes qui suivent consistant à :
a) charger une première colonne (VS1), qui est remplie d'un matériau échangeur de cations, avec les matériaux de départ dissous dans de l'acide minéral, lesquels contiennent les composés ¹⁷⁷Lu et ¹⁷⁶Yb selon un rapport massique allant d'environ 1:10² à 1:10¹⁰ ; remplacer les protons du matériau échangeur de cations contre des ions d'ammonium en utilisant une solution de NH₄Cl ; et rincer le matériau échangeur de cations de la première colonne (VS1) avec de l'eau ;
b) relier la sortie de la première colonne (VS1) à l'entrée d'une deuxième colonne (S1), qui est de la même manière remplie d'un matériau échangeur de cations ;
c) appliquer un gradient composé d'eau et d'un agent complexant, choisi parmi le groupe constitué de : α-hydroxybutyrate [HIBA], acide citrique, citrate, acide butyrique, butyrate, EDTA, EGTA et ions d'ammonium, en commençant à 100 % d'H₂O à 0,2 M d'agent complexant à l'entrée de la première colonne (VS1) ;
d) détecter la dose de radioactivité à la sortie de la deuxième colonne (S1) afin d'identifier une élution de composés ¹⁷⁷Lu ; et recueillir un premier éluat ¹⁷⁷Lu provenant de la sortie de la deuxième colonne (S1) dans un récipient (F2) ; et protoner l'agent complexant afin de le rendre inactif pour la complexation avec des ions ¹⁷⁷Lu ;
e) acheminer en continu l'éluat ¹⁷⁷Lu acide obtenu à l'étape d) vers l'entrée d'une troisième colonne (VS2), qui est remplie d'un matériau échangeur de cations, sachant que le matériau échangeur de cations est présent sous une forme protonée du fait du chargement en éluat ¹⁷⁷Lu acide ; remplacer les protons du matériau échangeur de cations par des ions d'aluminium en utilisant une solution de NH₄Cl ; et rincer le matériau échangeur de cations de la troisième colonne (VS2) avec de l'eau ;
f) relier la sortie de la troisième colonne (VS2) à l'entrée d'une quatrième colonne (S2), qui est remplie d'un matériau échangeur de cations ;
g) appliquer un gradient composé d'eau et d'un agent complexant choisi parmi le groupe constitué de : α-hydroxybutyrate [HIBA], acide citrique, citrate, acide butyrique, butyrate, EDTA, EGTA et ions d'ammonium, en commençant à 100 % d'H₂O à 0,2 M d'agent complexant à l'entrée de la troisième colonne (VS2) ;
h) détecter la dose de radioactivité à la sortie de la quatrième colonne (S2) afin d'identifier une élution des composés ¹⁷⁷Lu ; et recueillir un deuxième éluat ¹⁷⁷Lu provenant de la sortie de la troisième colonne (S2) dans un récipient (F3) ; et protoner l'agent complexant afin de le rendre inactif pour la complexation avec des ions ¹⁷⁷Lu ;
i) charger une cinquième colonne (S3), qui est remplie d'un matériau échangeur de cations, en acheminant en continu l'éluat ¹⁷⁷Lu acide obtenu à l'étape h) à l'entrée de la cinquième colonne (S3) ; laver l'agent complexant avec un acide minéral dilué ; éliminer des traces d'ions métalliques étrangers de la solution ¹⁷⁷Lu en lavant le matériau échangeur de cations de la cinquième colonne (S3) avec un acide minéral à des concentrations différentes comprises dans la plage allant d'environ 0,01 à 2,5 M ;
j) éluer les ions ¹⁷⁷Lu de la cinquième colonne au moyen d'un acide minéral concentré allant d'environ 1 M à environ 12 M ; recueillir l'éluat ¹⁷⁷Lu à pureté élevée dans un dispositif d'évaporation et éliminer l'acide minéral par évaporation.

11. Procédé selon l'une quelconque des revendications précédentes, **caractérisé en ce que** le procédé est effectué dans une cellule chaude de la classe C selon les directives GMP (good manufacturing practice - bonnes pratiques de fabrication) de l'UE.

12. Utilisation d'une colonne de chromatographie par échange de cations comprenant une résine échangeuse de cations en tant que phase stationnaire et un agent complexant en tant que phase mobile servant à fabriquer des composés ¹⁷⁷Lu à pureté élevée sans porteur de l'ordre du milligramme à partir d'une matrice ¹⁷⁶Yb de l'ordre du gramme, sachant que la colonne est remplie d'un matériau échangeur de cations, et que les protons du matériau échangeur de cations ont été remplacés par des ions d'ammonium en utilisant une solution de NH₄Cl ;
gradient composé d'eau et d'un agent complexant, choisi parmi le groupe constitué de : α-hydroxybutyrate [HIBA], acide citrique, citrate, acide butyrique, butyrate, EDTA, EGTA et ions d'ammonium, en commençant à 100 % d'H₂O à 0,2 M d'agent complexant, est appliqué à l'entrée de la colonne ; et
une dose de radioactivité est détectée à la sortie de la colonne afin d'identifier une élution des composés ¹⁷⁷Lu.
